# EUROPEAN PATENT SPECIFICATION

(11) **EP 4 333 763 B1**
(45) Date of publication and mention of the grant of the patent: **24.12.2025**
(21) Application number: 22728353.8
(22) Date of filing: 04.05.2022
(51) Int. Cl.: A61B 90/00, A61B 34/20, A61B 5/00

(54) **AUGMENTED REALITY HEADSET AND PROBE FOR MEDICAL IMAGING**
HEADSET MIT ERWEITERTER REALITÄT UND SONDE FÜR MEDIZINISCHE BILDGEBUNG
CASQUE DE RÉALITÉ AUGMENTÉE ET SONDE POUR IMAGERIE MÉDICALE

(30) Priority: 04.05.2021 GB 202106355
(43) Date of publication of application: 13.03.2024
(73) Proprietor: Arspectra Sarl, 3895 Foetz (LU)
(72) Inventor: SPAAS, Cedric, 9051 Gent (BE); ANNAIYAN, Arun, 1750 Luxembourg (LU); PEREZ-PACHON, Laura, 1880 Luxembourg (LU); LEGROS, Arnaud, 9123 Schieren (LU)
(74) Representative: Gill Jennings & Every LLP
(86) International application number: PCT/EP2022/062025
(87) International publication number: WO 2022/233961

(56) References cited:
- WO-A1-2015/154187
- WO-A1-2015/164402
- US-A1- 2018 270 474
- US-A1- 2020 383 577

## Description

### FIELD

The present invention relates to an augmented reality system for use in medical procedures.

### BACKGROUND

Cancer surgery consists of the removal of tumours. To ensure complete removal, surgeons excise a portion of healthy tissue surrounding the tumour. Complete removal is key to prevent tumour recurrence, which is associated with increased surgical costs and morbidity and mortality rates. In order to ensure complete removal tumours need to be correctly identified and accurately mapped.

Current techniques for tumour identification and mapping include:
- Biopsy, i.e., obtaining tissue samples using a needle for their posterior pathological analysis in the lab;
- Patient scanning, i.e., the tumour margins are assessed on medical images of the patient;
- Thermal imaging, i.e., cancer detection is made using infrared thermal imaging;
- Raman spectroscopy, i.e. a technique that allows analysing the chemical composition of biological tissues and has been extensively used for cancer screening, diagnosis, and intraoperative surgical guidance in the past ten years.

Raman spectroscopy for tumour identification and mapping is a powerful technique as it allows surgeons to distinguish between healthy and cancerous tissue during surgery while being less invasive than biopsies. However, known Raman techniques present a key limitation as the surgeon needs to take several measurements using the probe to obtain a complete map of the cancerous tissue as the area of tissue analysed at a time is very small (around 1mm). This means that the surgeon must recall multiple locations of the cancerous tissue determined by the probe measurements during surgery, or mark the detected areas of cancerous tissue using physical markers such as stickers. The first method leads to reduced accuracy, whilst the latter results in longer surgery times due to the surgeon having to stop and place the physical markers in the correct position.

A further solution to this problem is to observe the location of the cancerous tissue on images of the patient body displayed on monitors in the surgery room. However, the surgeon must then match this information to the actual patient's body before them. This can lead to reduced surgery accuracy as this matching is prone to human error relying on the surgeon's ability to apply the position shown on the monitor to the patient. In addition, this can lead to long surgery times as the surgeon has to constantly look away from the patient to the monitor.

The present invention is directed at solving the above described problems associated with current procedures.

US 2018/0270474 A1 discloses a registration process. The registration process obtains the position of a patient or target object, a wearable imaging and display system, and a handheld probe as a peripheral coupled to the interface.

Next, the system acquires pre-operative imaging data. Next, a 3D model is created based on the pre-operative imaging data. In the next step, the position of the patient is tracked intra-operatively using technique, such as fiducial markers for example. Next, a transformation matrix is calculated between the pre-operative image space and the intra-operative object space (i.e. patient space). Continuing, the pre-operative image data is registered to the intra-operative object space (i.e. patient space). Next, the intra-operative imaging data is acquired from the imaging system, such as fluorescence or color imaging for example. Continuing, the position of the wearable imaging and display system is obtained, using technique, such as optical tracking or magnetic tracking). Next, the transformation matrix between the intra-operative imaging space (i.e. wearable imaging and display system) and the intraoperative object space (patient space) is calculated. The intra-operative imaging space (such as fluorescence image data) is then registered to the intra-operative object space (i.e. patient space). In addition, the process acquires handheld device imaging or sensing data, such as ultra-sound fiber microscope, and Raman spectroscopy for example. In addition, the position of the hand-held probe, such as an ultrasound fiber, a microscope, and Raman spectroscopy probe is tracked. Next, a transformation matrix is calculated between the hand-held imaging/sensing probe image space and the intra-operative object space (i.e. patient space). Continuing, the hand-held device image space (i.e. ultrasound or microscope) is registered to the intra-operative object space (i.e. patient space). Finally, the co-registered image data is presented on a display of the wearable imaging system.

### SUMMARY OF INVENTION

According to a first aspect there is provided an augmented reality (AR) system for obtaining and displaying measurements from a patient during a medical procedure, the system comprising: an AR headset comprising a near eye display for displaying the measurements overlaid in a surgeon's view of the patient's tissue at a position with respect to the patient to which they were collected; at least one sensor for tracking the location of the patient; a memory for storing the measurements and their associated positions; and a processor; wherein the system is configured to perform the following steps during the medical procedure: (a) receiving measurements collected from the patient's tissue, wherein the measurements are collected by a probe being positioned adjacent to various regions of the patient's tissue; (b) tracking, the probe's position whilst the probe is collecting measurements to determine a position at which the measurements are collected; (c) simultaneously to performing steps (a) and (b): tracking, using the at least one sensor, a position of a biological landmark on the patient such that a relationship can be determined between the position of the biological landmark and the position at which the measurements are collected; (d) displaying on the near eye display the measurements at a position, such that the measurements are aligned in the surgeon's view of the patient's tissue at the position with respect to the patient to which they were collected, throughout the medical procedure, by: continuously tracking the position of the biological landmark throughout the medical procedure to obtain its current position; updating the position of the measurements on the display based on the biological landmark's current position.

In this way, as the biological landmark's position is known at the same time as when the probe measurement is obtained a relationship between their relative positions can be determined. Therefore, through continually tracking the position of the biological landmark the measurement, when displayed on the display at a later point in time, can be overlaid on the display at the correct position with respect to where it was obtained from the body of the patient.

As biological landmarks, are used for the tracking there is no need to place physical markers on the patient or surrounding the patient resulting in a physical marker-less procedure. This is advantageous as biological landmarks will not fall off the patient as physically attached markers may do. In addition, the surgeon does not need to stop and attach markers to the patient, thereby potentially reducing the duration of the surgery or the harm and risk caused to the patient.

The surgeon has the view of the measurements on the augmented reality display directly in their line of sight of the patient as the patient's body is tracked in real time. In other words the measurements are overlaid on their real world view of the patient at the position the measurements correspond to. This means they do not have to continually look away from the patient's body to look at an external monitor. This also reduces the error associated with the surgeon subjectively translating what they see on the monitor to the actual body of the patient.

The step of updating the position of the measurements on the display based on the biological landmark's current position involves using the relationship between the position of the biological landmark and the position at which the measurements are collected.

The position at which the measurements are collected is the position of the patient where each of the measurements are collected. In other words, the point on the patient from which the probe has taken the measurement. The relationship may be between the position of the biological landmark and the position at which each measurement is taken determined through tracking the position at which each measurement is taken. The term adjacent the patient tissue may be interpreted to mean next to, on, or in the patient tissue depending on the nature of the probe and the measurement to be taken.

Preferably, the biological landmark is one or more features of the biological landmark. In this way, the tracking is of the one or more features of the biological landmark. For instance, this may involve tracking, using the at least one sensor, a position of the one or more features of the biological landmark on the patient such that a relationship can be determined between the position one or more features of the biological landmark and the position at which the measurements are collected. It may further comprise continuously tracking the position of the one or more features of the biological landmark throughout the medical procedure to obtain its current position; and updating the position of the measurements on the display based on the one or more features of the biological landmark's current position.

The one or more features of the biological landmark may comprise edges or points of intersection of the biological landmark. These may be elements of the biological landmark that can be identified. Advantageously, the points of intersection or edges may provide an easy to identify reference point on the biological landmark. This features may be extracted from raw data obtained by the sensor.

The sensor may track the location (i.e. position and orientation) of the patient. The current position is the position of the biological landmark at the present moment in time, owing to the fact that the position of the patient may change throughout the procedure due to movement of the patient and movement of the AR headset with respect to one another.

The method may further comprise creating a virtual map of the measurements, wherein displaying on the near eye display the measurements comprises displaying the virtual map. The virtual map may provide the surgeon with a visual guide as to regions of the patient tissue that are cancerous. The virtual map may be a spatial map of the measurements providing a view to the surgeon that is intuitive and easy to interpret.

The virtual map may be a colour coded data map with regions of different measurement values indicated by different colours. For instance, cancerous tissue may be indicated in a different colour to regions of non-cancerous tissue. Alternatively, or in addition, the amount of cancerous tissue may be indicated by to the colour of the virtual map. Regions with a higher percentage of cancerous tissue may have a different colour to regions with a lower percentage of cancerous tissue.

Preferably, tracking the position of the biological landmark on the patient may further comprise: illuminating a region of the patient using light of a specific wavelength and detecting by the sensor a fluorescent signal emitted by the biological landmark in response to the illuminating. Prior to the surgery the patient may be injected with a fluorescent dye into their venous system. The dye may then accumulate in the biological landmark of interest. For instance, this may be the blood vessels of the patient. Advantageously, by using a fluorescent dye internal regions of the patient can be tracked that are located within the patient's body. This is advantageous as it allows the tracking point to be as close to the point of interest as possible. In addition, it does not require a surgically invasive procedure of inserting a physical marker close to the detection position. As a patient body is not fixed in position and will move throughout the procedure, for instance when breathing, by tracking the biological landmark the marker will move with the patient body thereby improving the tracking, and thereby the overlay of the measurements on the display.

The fluorescent marker may be any known fluorescent dye or contrast agent. For instance, it may be indocyanine green (ICG). Illuminating the patient may involve using a near-infrared (NIR) light source. The light source excites the molecular ligands in the biological landmark, which in response emits light with a specific wavelength. A camera sensitive to this range of the light spectrum may be used to detect the light. The detected light may be in the form of raw data that may be used to form an image which clearly denotes the biological landmark. The wavelengths of NIR may be selected depending on the dye being used. The fluorescence signal is used for positional tracking purposes rather than itself being indicative of a particular medical measurement. The NIR camera and NIR light source may be located on the AR headset. In other arrangements, the NIR camera and/or NIR light source may be located separate from the AR headset. Alternatively, any other type of fluorescent marker may be used, such as methylene blue, alanine blue, and others which may be sensitive to light in the visible spectrum. The camera and light source may be chosen based on the type of contrast agent used in either the visible, NIR or IR wavelengths.

The AR system may further comprise a probe. Preferably, the probe is a Raman probe.. Advantageously, Raman spectroscopy provides a non-invasive procedure for identifying and mapping cancerous tissue. By presenting these measurements on the near eye display the surgeon can view them at the correct position with respect to the patient in their direct line of sight. The probe may be a handheld probe. For instance, it may be a handheld tool for collecting measurements from the patient. A Raman probe may comprise a diode laser for exciting the tissue and a sensor for detecting the Raman scattering measurements from the tissue. However, other arrangements of Raman probes may be used.

Alternatively, the probe could be a temperature probe capable of acquiring temperature measurements from a patient. The temperature measurements may be displayed on the display as a virtual map at the correct position in the surgeon's view relative to where they were acquired. In other arrangements, the probe may be an electromagnetic probe. For instance, the electromagnetic probe may be an electromagnetic pulsation probe. The electromagnetic pulsation probe may be used to take measurements from, or give impulses to a patient's brain in an invasive procedure. Alternatively, any other type of probe may be used, such as for instance a PH probe.

When the AR system comprises the probe it may further include performing the step of collecting the measurements using the probe. However, it would also be understood that the probe may instead be considered separate to the AR system.

The medical procedure may be a surgical procedure. Specifically, it may be cancer surgery. This may be surgery to remove cancerous tissue, such as a tumour, from the patient. The present invention allows the measurements to be collected during the surgical procedure whilst enabling them to be visualised by the surgeon throughout said procedure. Alternatively, in other arrangements the measurements may be collected prior to the surgical procedure for later display during the surgical procedure. The measurements may act as a guide for the surgeon throughout the surgery.

Preferably, the at least one sensor may comprise an RGB camera. For instance, the RGB camera may obtain images of the biological landmark throughout the procedure. In some arrangements there may be provided a single RGB camera. In other arrangements, there may be multiple RGB cameras. In other arrangements the camera may be one or more near infrared (NIR) or infrared (IR) cameras. Alternatively, multiple cameras can be combined, or multispectral and hyperspectral sensors can be used. The selection of the nature of the camera will depend on the nature of the biological landmark that is being tracked. Having a single camera enables 2D images to be produced (unless moved to register and create a 3D volume), whereas multiple cameras enables the generation of 3D images/models to be produced.

In addition, the headset may comprise a depth sensor. For instance, this may be a time of flight sensor. Alternatively this may be a stereo cameras, LIDAR, RADAR, hyperspectral cameras, or combinations of sensors, or any other known type of sensor capable of measuring distance and scale. The depth sensor is capable of measuring the distance from the headset to the patient. In this way, the position of the headset relative to the patient can be determined throughout the procedure thereby improving the accuracy of positioning the virtual map on the display. For instance, the depth sensor may estimate the position and/or orientation (pose) of the headset relative to the patient to be determined throughout the procedure. This may be through generating point clouds or depth images of the surgical site. This may aid in providing a transformation between the patient coordinates and the headset coordinates. The depth sensor may generate a depth map or point cloud of the biological landmark. As the positions of the measurement of the probe may be 3D data, in order to determine a relationship with the position of the biological landmark (e.g. features) the 3D position of the biological landmark (e.g. features) need to be determined. This can be achieved by obtaining a depth map or point cloud.

Continuously tracking the position of the biological landmark throughout the medical procedure to obtain its current position may further comprise: capturing, by the sensor, images of the patient; extracting one or more features from the images of the biological landmark; determining a correspondence between the one or more features across consecutive frames of the images; estimating the motion of the one or more features across consecutive frames of the images using a transformation matrix. The transformation matrix is preferably already calculated and based on calibration parameters of the sensors. The extracting one or more features may be by applying an algorithm to extract said features. This may be from raw image data.

The images of the patient may be raw data, such as the emitted light from fluorescence. Successive images over time may form a series of image frames. The features may be 2D or 3D features depending on the nature of the raw image data that is obtained. Robust algorithms may be used to identify the 2D/3D features. This identification may be based on, for example, the size, shape and colour intensity of the biological landmarks. The correspondence between the features across consecutive frames of images may be used to track the features.

Continuous calculation of the transformation is performed throughout the procedure to allow for real-time tracking during surgery.

The method may further comprise, prior to calculating the transformation matrix, applying an optimisation procedure to remove noisy or incomplete data. This may involve using global optimization.. Alternatively, or in addition, an outlier rejection algorithm or optimization filter may be used. For instance, optimized Random sample consensus (RANSAC) or Iterative closest point algorithms. Advantageously these techniques can help to remove noise and speckle thereby reducing false matches that may be caused by tracking drifts due to noisy data. For example due to the illumination of the environment.

Estimating the motion of the features across consecutive frames of images by using a transformation matrix may further comprise: applying a rigid or non-rigid registration. As the patient's body is a dynamic object (e.g. due to respiration movements or deformation of soft tissues), displacements of the 2D/3D features across several frames may occur. By calculating and then using the transformation matrix the motion of the 2D/3D features can be estimated across consecutive frames. Non-rigid registration and elastic deformation correction may be used to estimate the position of the 2D/3D features in real-time. While doing the non-rigid body estimation, the region of interest can shrink or expand resulting in dynamic raw data. For this reason, elastic deformation corrections may be necessary. This can allow accurate matching between image frames despite the changes and movement to the patient's body.

Due to the dynamic nature of the system, the shape of the features can also change across frames, which could result in non-uniformities in the images. In some arrangements an image gradient operation on the 3D data may be used to determine the displacement between the location of a feature between frames. For this, an acceptance criterion may be defined and a threshold associated to it. Once the threshold is reached the coordinate system of the probe is registered with a coordinate system of the patient.

As outlined above, the one or more features of the biological landmark may comprise edges or points of intersection of the biological landmark. Advantageously, the points of intersection or edges may provide an easy to identify reference point on the biological landmark. This may improve the accuracy of the tracking of the landmarks between frames. Alternatively, rather than features of the biological landmark being used the entire structure of the biological landmark may be tracked. For instance, if it is a skin mark, such as a birth mark, the features may be the shape of the biological landmark.

Preferably, the biological landmark may comprise blood vessels of the patient. As the patient blood vessels are located throughout the body of the patient they enable tracking to be performed at any location throughout the patient's body. The blood vessels may be illuminated by NIR light to enable their sub-surface detection. Alternatively, the biological landmark may be at least one of Lymph nodes of the patient, nervous systems of the patient, organ surfaces of the patient

Alternatively, other biological landmarks may be used. For instance, a skin mark, a mole, a birthmark, a joint, muscle, an organ, or any other region of patient tissue. Alternatively, the biological landmarks may include anatomical landmarks, such as the corner of the eyes or the suprasternal notch.

In some arrangements multiple biological landmarks may be used for tracking. For instance, multiple blood vessels may be used and each of their features tracked. In other arrangements, multiple different types of biological landmarks may be used. For instance, both blood vessels and skin marks. Increasing the number of biological landmarks that are used may increase the accuracy of the patient tracking.

The step of tracking the probe's position may comprise detection, via the sensor, a marker on the probe and calculating a position of a tip of the probe representing the position at which the measurement is collected. In this way, the probe itself can be tracked with the position of the tip of the probe indicating the position that the measurement was taken. Thus, the position at which the measurement was taken can be determined with high accuracy. A relationship between the position of the probe tip and the marker is calculated according to the known probe geometry. There may be a single marker or a plurality of markers.

The probe markers may be a visible reference such as a geometrical shape on the probe. Alternatively, the probe markers may be a marker that actively emits electromagnetic radiation.

The probe marker may be detected by a tracking system on the headset. For instance, the one or more sensors may track the probe marker. For instance, this may be an electromagnetic sensor. The sensor may be an infrared camera, or an RGB camera for detecting an image of the marker. The sensor may detect an image of the probe articificial marker such that image pattern recognition carried out to identify the position of the marker. This may use for example, using Aruco, April tag, or using a known pattern. In other arrangements, machine learning algorithms may be used for tracking the position of the probe. The machine learning algorithms (e.g. AI) may be trained to track the position of the probe.

Alternatively, the tracking system may be external to the headset thereby performing external tracking. In this arrangement, the position of the headset would also be tracked in addition to the probe position.

In other arrangements, the geometry of the probe may be tracked through using a plurality of cameras in stereo configuration. This may enable the position of the probe to be tracked through comparing the images of the probe from each of the cameras and through knowing the probes geometry. In further arrangements, a sensor located on the probe may be responsible for tracking the probes position. For instance, this may be estimated through using odometry. For instance, the sensor located on the probe may be an inertial measurement unit (IMU). Advantageously, using an IMU may improve the accuracy of the tracking and also can anchor the virtual map in a fixed position relative to the real world.

Alternatively, the probe may be tracked through using an electromagnetic generating device (e.g. a mat) placed on the table below the patient. In this arrangement, the probe and headset comprise magnetic markers placed on them and the AR system comprises an device configured to generate an electromagnetic field. In this way, the magnetic field generated by said device detects the movement of these small magnetic units within the electromagnetic field thereby tracking their motion to obtain their spatial localisation. In this arrangement, a RGB camera used to track the probe may not be required. Preferably, the step of tracking the probe's position may comprise detecting, a change in magnetic field caused by the movement of a magnetic marker on the probe, wherein the change in magnetic field identifies the position of the probe.

The measurements may be for indicating regions that indicate cancerous tissue. Each measurement may comprise an intensity with the location of the probe at the point of acquiring the measurement associated with it. The intensity may indicate that the tissue where the measurement is acquired is cancerous. This intensity may be shown in the virtual map to indicate the regions of cancerous tissue.

The relationship between the position of the biological landmark (e.g. the one or more features) and the position at which the measurements are collected may be determined by transforming both positions in the same coordinate system.

Specifically, transforming the position of the features of the biological landmark and the position at which the measurements are collected into a common coordinate system. By registering the coordinate system of the measurements with a coordinate system of the biological landmarks at the instance when the measurements were taken the relative positions between the biological landmark and the measurements can be easily determined in the same frame of reference thereby enabling determination of the relationship. In this way, each point where a feature or a measurement is, is represented by a coordinate vector. Advantageously, through this registration the measurements (or virtual map) are correctly aligned with the patient's body when viewed by the surgeon. This registration is continually carried out throughout surgery. This may involve transforming the coordinate systems into a common coordinate system to ensure that the virtual map and the images of the biological landmarks can be compared to each other and thereby be correctly aligned on the display based on the determined relationship. This may be through transforming the coordinates of the measurements in the headset frame of reference into the coordinates of the biological landmark frame of reference such that the relative positions can be determined.

Preferably, displaying on the near eye display the measurements at a position, such that the measurements are aligned in the surgeon's view of the patient's tissue at the position with respect to the patient to which they were collected, throughout the medical procedure, may further comprise: tracking a position of the surgeon's eyes and adjusting the position of the measurements on the near eye display based on the current position of the surgeon's eyes. Advantageously, this means that the positioning of the virtual map on the display may be more accurate by taking into account the position of the surgeon's eyes. The position may be tracked throughout the medical procedure to ensure real time adjustment.

Throughout the medical procedure the position of the wearer's eyes will not be fixed with their view and gaze varying throughout the procedure. The accuracy of the positioning of the generated image over the wearer's view can be improved by the continuous determination of the position of the wearer's eyes and their position relative to the headset. The eye tracking sensor may also determine the focus of the wearer's eyes, i.e. the position of where they are focusing on at any particular time. In this way the virtual map can be displayed such that it is displayed in focus at all time in the wearer's view of the patient.

The eye tracking sensors may be a plurality of eye tracking sensors. For instance, there may be a single sensor that tracks each eye. Alternatively, a single tracking sensor may track the position of both eyes. The eye tracking sensors may comprise an IR light source and detector that scans the position of each eye to determine its position. The light source may be in the form of an LED or laser. Alternatively, or in addition, the eye tracking sensor may be an electric potential eye tracking sensor. The electric potential eye tracking sensor uses electrodes placed around the eye to measure the movement of the eye.

The processor may receive the position of the wearer's eyes. Preferably, the processor is further configured to obtain the position of the wearer's eyes through obtaining the interpupillary distance of the wearer's eyes.

The near eye display may be a single display. Alternatively, the near eye display may be two displays, one display for displaying an image for each eye. The near eye display may be a waveguide. Alternatively, the display might be a beam splitter display, or a laser reflection display. The display may utilise mirrors to project the image into the wearer's view. The display may be made of glass and/or plastic. In this way it is transparent. The near eye display may be alternatively a lens. Or the near eye display may be a beam that projects the image into the eyes of the wearer such that the image is displayed on the retina. In this way the near-eye display may be a virtual retinal display.

The device may further be configured to perform a calibration procedure to calibrate the AR headset. The calibration procedure may involve obtaining the relative positions of the sensors on the headset with one another. The step of displaying may be updated based on said calibration. The processor may determine the relative positions of the sensors, and the display(s) with respect to each other. The parameters of the sensors may also be determined. For instance, parameters for a pinhole camera model used by the algorithms, or parameters to correct the distortion introduced by the optics in front of the sensors may be determined. A further calibration is also carried out to determine the interpupillary distance (IPD) of the surgeon (i.e. the distance between their eyes). This may be obtained through taking measurements using eye tracking sensors to determine the IPD. Alternatively, this may be manually input into the software by the surgeon. The position of the surgeon's eyes with respect to the display may also be determined. This calibration ensures accurate overlay of the probe measurements taken with the correct position of the patient from where they were collected. The transformation matrix may comprise details of the calibration parameters obtained.

According to a comparative example, there is provided a method of using an augmented reality (AR) system to obtain and display measurements from a patient to a surgeon during a medical procedure, comprising the steps: (a) receiving measurements collected from a patient's tissue, wherein the measurements are collected by a probe being positioned adjacent to various regions of the patient's tissue; (b) tracking, the probe's position whilst the probe is collecting measurements to determine a position at which the measurements are collected; (c) simultaneously to performing steps (a) and (b): tracking, using the at least one sensor, a position of a biological landmark on the patient such that a relationship can be determined between the position of the biological landmark and the position at which the measurements are collected; (d) displaying on a near eye display of an AR headset worn by the surgeon the measurements at a position, such that the measurements are aligned in the surgeon's view of the patient's tissue at the position with respect to the patient to which they were collected, throughout the medical procedure, by: continuously tracking the position of the biological landmark throughout the medical procedure to obtain its current position; updating the position of the measurements on the near eye display based on the biological landmark's 'current position.

The augmented reality system of this aspect may be the augmented reality system of the above aspect.

According to a further aspect there is provided a computer program product comprising instructions which, when the program is executed by a computer, cause the computer to carry out the steps of: (a) receiving measurements collected from a patient's tissue, wherein the measurements are collected by a probe being positioned adjacent to various regions of the patient's tissue; (b) tracking, the probe's position whilst the probe is collecting measurements to determine a position at which the measurements are collected; (c) simultaneously to performing steps (a) and (b): tracking, using the at least one sensor, a position of biological landmark on the patient such that a relationship can be determined between the position of the biological landmark and the position at which the measurements are collected; (d) displaying on a near eye display of an AR headset worn by the surgeon the measurements at a position, such that the measurements are aligned in the surgeon's view of the patient's tissue at the position with respect to the patient to which they were collected, throughout the medical procedure, by: continuously tracking the position of the biological landmark throughout the medical procedure to obtain its current position; updating the position of the measurements on the near eye display based on the biological landmark's current position.

According to a further aspect there is provided a non-transitory computer readable medium, that when executed on a processor is configured to perform the above method.

### DESCRIPTION OF FIGURES

Figure 1 shows a schematic view of a prior Raman system for analysing the chemical composition of biological tissues;
Figure 2 shows a schematic view of an example augmented reality (AR) headset according to an embodiment of the present invention;
Figure 3 shows a schematic view of an example augmented reality (AR) system according to an embodiment of the present invention for obtaining and visualising measurements;
Figure 4 shows a flow chart of steps performed for obtaining measurements from a patient and displaying them accurately in the view of the user using the example AR system as shown in Figure 3; and
Figure 5 shows an example virtual map as displayed in the AR headset of Figure 2 obtained using the AR system of Figure 3.

### DETAILED DESCRIPTION

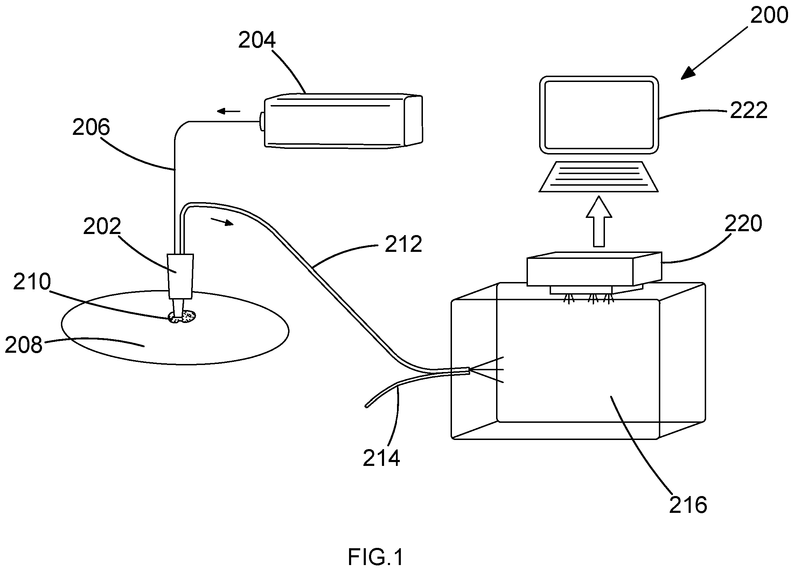

Figure 1 shows a prior Raman system 200 for analysing the chemical composition of biological tissues, such as to detect and map tumours. The system 200 comprises a diode laser 204 attached via a fibre optic cable 206 to Raman probe 202. The Raman probe 202 is also attached by fibre optic cable 212 to spectrograph 216. Spectrograph 216 may have a volume phase technology (VPT) grating coupled to a CCD camera 220. The CCD camera 220 is in communication with computer processing unit 222.

During the Raman spectroscopy procedure, the probe 202 is placed adjacent to the tissue 210 of the patient 208 to be analysed, such that a low power laser beam from the laser 204 is incident on the area of tissue 210 to be analysed, as can be seen in Figure 1. This light incident on the tissue causes the tissue to emit light through Raman scattering which is then detected by the probe 202 and sent to the spectrograph 216 (i.e., an instrument that detects and analyses incoming light according to its wavelength and records the resulting spectrum).

The output data of the spectrograph 216 is processed by the CCD camera 220 and computer processing unit 222. As cancerous tissue emits light within a certain spectrum, those values that are compatible with cancerous tissue can be converted into a graphical representation (i.e. a virtual map). This virtual map is aligned with an image of the patient's tissue 208, thus highlighting the areas of cancerous tissue. The image is merged with the virtual map are then displayed on a monitor that is set up in the operating room. The surgeon uses this visualization as guidance to locate the areas of cancerous tissue during surgery.

However, displaying the image on a monitor remote from the patient in the surgery room has disadvantages. The surgeon must match this information displayed on the monitor to the actual patient's body before them. This can lead to reduced surgery accuracy as this matching is prone to human error relying on the surgeon's ability to apply the position shown on the monitor to the patient. In addition, this can lead to long surgery times as the surgeon has to constantly look away from the patient to the monitor.

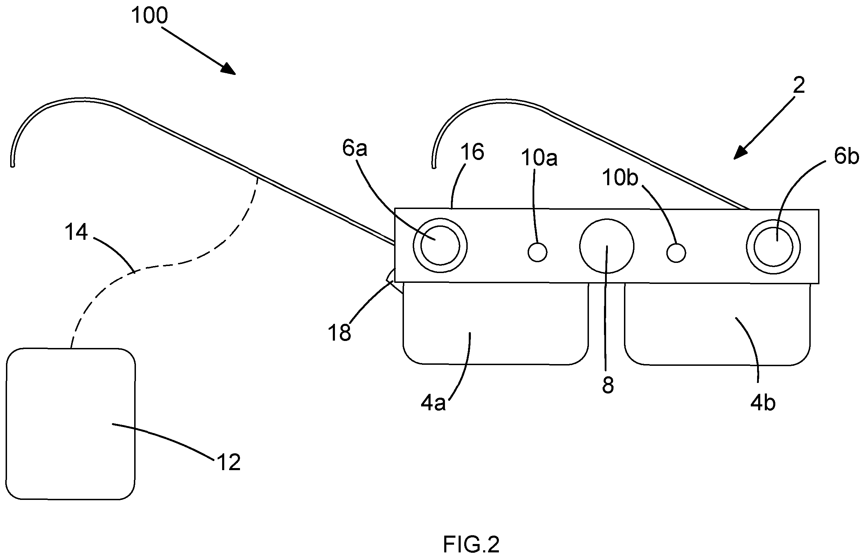

Figure 2 shows an augmented reality (AR) system 100 according to an embodiment of the present invention. The AR system 100 includes an AR headset 2 and a processor 12.

The augmented reality headset 2 has two displays a first display 4a and a second display 4b, the first display 4a for displaying an image to the right eye of the wearer of the headset and the second display 4b for displaying an image to the left eye of the wearer of the headset 2. The displays 4a and 4b are attached to a housing 16 of the headset 2.

Located on the housing 16 of the headset are two cameras 6a 6b. Camera 6a is located above the first display 4a, and camera 6b is located above the second display 4b. The cameras 6a 6b are capable of detecting near infrared (NIR) light, and RGB light (i.e. multispectral cameras). These are shown as being two cameras however, any number of cameras may be used. In addition, separate cameras may detect the visible and NIR light.

Also located on the housing 16 is a light source 8. The light source 8 is a NIR light source configured to emit NIR light. The light source 8 is located between the cameras 6a and 6b, although it could be located at any position on the AR headset 2. Alternatively, the light source 8 may be located external to the AR headset 2 (or for certain types of set ups they might not be needed at all).

Two depth sensors 10a and 10b are located on the housing 16 of the headset 2. The depth sensors are time of flight sensors configured to determine a depth to an object from the headset 2, although any type of depth sensor may be used.

The headset 2 further includes an eye tracking sensor 18. The eye tracking sensor is located on the side of the headset that faces the head of the wearer. The eye tracking sensor is configured to determine the position of the eyes of the wearer of the headset 2. The eye tracking sensor 18 may also determine in which direction the eye of the wearer is looking.

The processor 12 is located external to the AR headset 2. The processor may be a processor of a computer or other data processing device. The AR headset 2 is connected to the processor 12 through cable 14. The cable 14 is for sending signals between the headset and the processor 12. For instance, the data obtained from the cameras 6a 6b, eye tracking sensor 18, and depth sensors 10a 10b may be sent through cable 14 to the processor 12. The cable 14 also is for sending communication signals between the processor 12 and the headset 2 to control the camera 6a 6b, depth sensor 10a 10b, light source 8, and eye tracking sensor 18 to perform their functions.

The AR system 100 may be used in combination with a Raman probe (not shown) during a surgical procedure, such as a procedure to remove cancerous tissue. The AR system can be used to obtain measurements of cancerous regions of a patient's tissue and to display the measurements on the displays 4a 4b to the surgeon in their line of sight thereby providing guidance during the surgery for the surgeon.

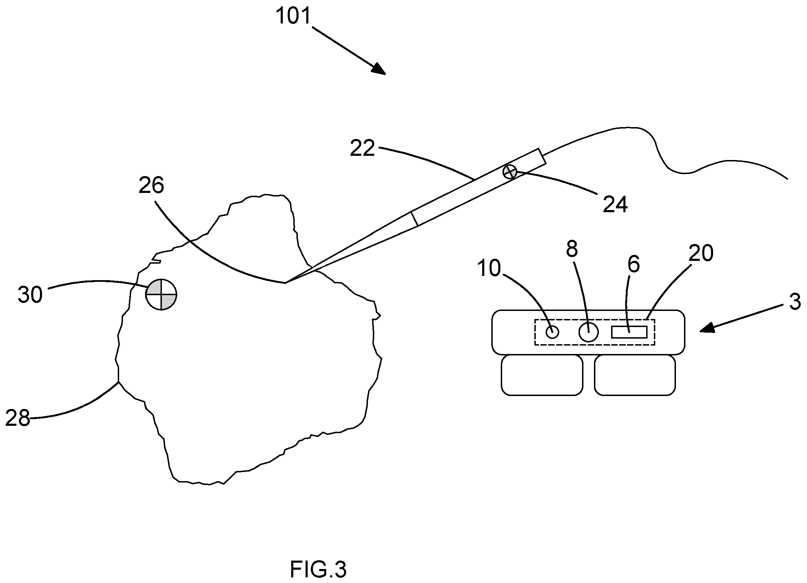

Figure 3 shows a further example augmented reality (AR) system 101 according to an embodiment of the present invention. The AR system 101 of Figure 3 has AR headset 3. AR headset 3 has the same features as AR headset 2 of Figure 2 having a sensor suite 20 with multiple cameras 6, a light source 8, and multiple distance sensors 10 (Although multiple cameras and distance sensors are not explicitly shown in Figure 3).

AR system 101 further includes Raman probe 22. The Raman probe 22 has the structure as described in relation to Figure 1 above, and is connected to the processor through a spectrograph (not shown). The probe consists of a measurement tip 26 which is the region of the probe that collects measurements. The probe 22 also includes a marker 24 that can be used for tracking its position relative to the patient and the AR headset 3.

Although processor 12 is not shown in Figure 3, AR system 101 has a processor in communication with the AR headset 3 and probe 22 for carrying out the processing steps.

The probe 22 is shown in Figure 3 adjacent to a region of patient tissue 28. The patient tissue 28 is the region from which measurements are to be collected by the probe 22, e.g. a suspected cancerous site. Located on the patient tissue 28 is an biological landmark 30 used to extract features for tracking the position of the patient, as will be described in detail below.

One particular example method of use of the AR system of Figure 3 will now be described. This example procedure is directed to obtaining measurements from the brain's cortex with the biological landmark 30 being the blood vessels on the brain surface in which the fluorescent marker has collected. However, it would be understood that this method may be applicable to any part of the patient's body. In this example procedure the patient is initially injected with a fluorescent dye, such as into their venous system. The dye accumulates in the blood vessels, or tissue of the patient, and can act as the biological landmark 30 as shown in Figure 3 through emitting light through fluorescence.

During the surgical procedure the surgeon wears the AR headset 3 and places the Raman probe 22 next to the patient tissue 28 to be analysed. The RGB camera 6 detects the probe marker 24 when the probe 22 is in the line of sight of the RGB camera 6. The processor then implements pattern recognition algorithms to estimate the marker's 24 pose (i.e. its position and orientation). This can then be used to estimate the position of the probe tip 26, i.e. where the Raman measurements are collected. As the processor knows the geometry of the probe 22 a transformation that accounts for the probe's geometry can be used to estimate the position of the probe tip 26 from tracking the probe marker's position. The headset coordinate system is then set. Within the headset coordinate system are specified positions of the probe's tip that match positions of areas of cancerous tissue.

The surgeon places the Raman probe 22 next to the patient in order to acquire measurements from the patient tissue. The collected measurements are analysed and those that indicate cancerous tissue can be stored. The measurements are stored along with the corresponding position of the probe 22 through tracking via marker 24. These stored measurements may have an intensity value associated with them and positional coordinates stored as (x, y, z). This allows a 3D virtual map to be generated.

At the same time as the above, the NIR light source 10 illuminates the patient tissue causing the fluorescent dye that has been injected into the patient to emit light through fluorescence. The location of injection may be chosen such that the dye accumulates in a particular region of the patient, such as specific blood vessels near the area of interest for probe measurements. These blood vessels can act as the biological landmark 30 used to extract features for tracking the patient. The fluorescence light emitted from the blood vessels is detected by the NIR camera 6 as a series of images. 2D or 3D features of the biological landmarks are extracted from these raw data images. The patient coordinate system is set and the 2D/3D features are saved in it

The relative position between the features and the probe measurement positions can be calculated to determine a relationship between their positions that can be used throughout the procedure to update the position of the virtual map of the measurement results on the display through tracking the positions of the features. This may be through transformations between the patient and the headset coordinate systems .

The position of the features (of the biological landmarks) across consecutive frames (i.e. their motion) is estimated (as will be described in further detail below) during the tracking process.

As outlined above, a virtual map is generated from the probe measurements. The virtual map indicates regions of tissue that are cancerous. Registration is then carried out such that the patient coordinate system is mapped to the headset coordinate system. In this way, the virtual map can be displayed on the AR headset display at the correct position with respect to the patient. This enables the surgeon to perform an operation procedure with the virtual map indicating cancerous tissue (for instance) clearly aligned with the patient thereby acting as a guide overlaid on the surgeon's view of the patient at the correct position. This is achieved through continually tracking the position of the features through continually detecting the NIR fluorescent signal from the biological landmarks (e.g. blood vessels) throughout the procedure, thereby forming a series of image frames.

In addition, eye tracking may also be used to further orientate the virtual map on the display at the correct view for the surgeon. One or more eye tracking sensors may track the coordinates of the patient's eyes. Through transforming the coordinates of the surgeon's eyes into a headset coordinate system the position of the virtual map may be updated on each display

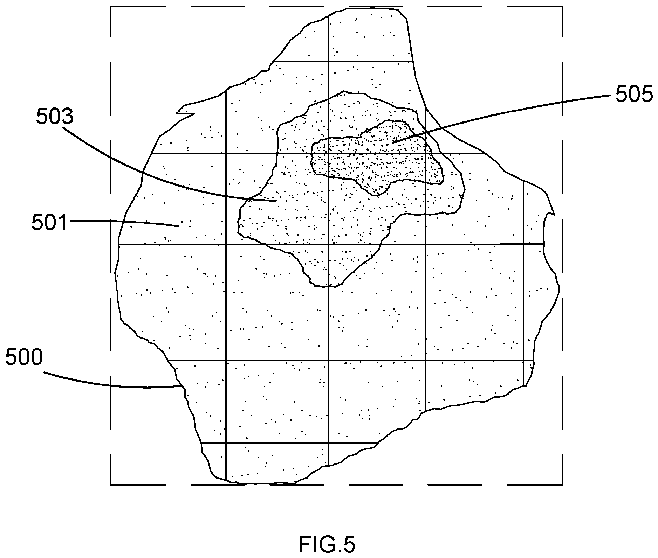

Figure 5 shows an example virtual map 500 as discussed above as displayed on the AR headset obtained using the AR system of Figure 3. The virtual map may be overlaid on the displays 6a 6b at the correct position relative to the surgeon's view of the patient at the position where the measurements were taken. Virtual map 500 shown in Figure 5 has three regions having different intensities indicated by the colour, or shade, of the regions of the map. The intensities indicate the intensity of the measurements recorded. For instance, in the examples described herein a higher intensity (i.e. darker region) may indicate a higher concentration of cancerous tissue. Region 501 has the lowest intensity and indicates a region with no cancerous tissue. Region 503, located within region 501, has a higher intensity than region 501 and indicates a small level of cancerous tissue. Region 505, located within region 503, has a higher intensity than region 503 and region 501 and indicates a high level of cancerous tissue. For instance, region 505 may indicate a tumour site.

The virtual map shown in Figure 5 is illustrated as a 2D map, which is the way in which it will be viewed by the surgeon when displayed on displays 4a 4b. However, the virtual map may actually be comprised of a 3D map of the measurements obtained from the tissue site which a 2D projection from said 3D map is displayed on the display. The virtual map displayed on each display 4a 4b may be a different projection of said 3D map. This takes into account the different positions of the surgeon's eyes.

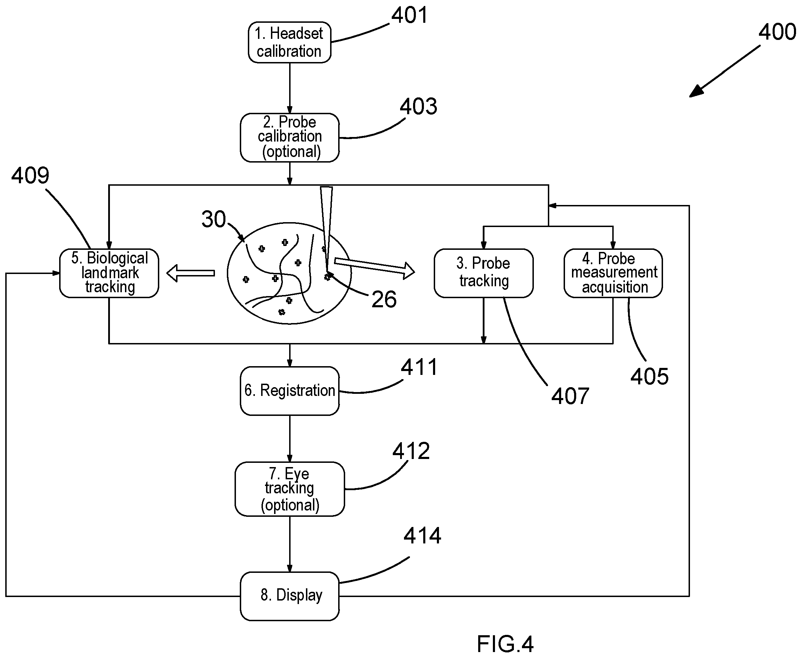

Figure 4 shows a flow chart of the method 400 described above performed for obtaining measurements from a patient and displaying them accurately in the view of the user of the AR system as shown in Figure 3.

At step 401 a calibration procedure is carried out to calibrate the AR headset. This may comprise calibration of the sensors on the headset to take into account their relationships with one another. The processor determines the relative positions of the sensors 6, 8, 10, and the display(s) 4 with respect to each other. The parameters of the sensors may also be determined. For instance, parameters for a pinhole camera model used by the algorithms, or parameters to correct the distortion introduced by the optics in front of the sensors may be determined. A further calibration is also carried out to determine the interpupillary distance (IPD) of the surgeon (i.e. the distance between their eyes). This may be obtained through taking measurements using eye tracking sensors to determine the IPD. Alternatively, this may be manually input into the software by the surgeon. The position of the surgeon's eyes with respect to the display may also be determined. This calibration ensures accurate overlay of the probe measurements taken with the correct position of the patient from where they were collected. The eye tracking related calibrations can be carried out continuously throughout the use of the device.

At step 403 an optional probe calibration is performed. This may enable the geometry of the probe to be determined such that the position of the tip of the probe 26 can be determined relative to the position of the probe marker 24.

At step 405 the Raman probe is placed adjacent to the patient tissue and measurements are acquired. The probe may determine measurements from tissue areas of interest, such as detecting areas of cancerous tissue. The measurements can be taken continuously by the surgeon simply placing the probe next to the patient tissue, or by the surgeon pressing a button on the probe while pointing at the area of interest to activate the acquisition of a measurement.

At step 407 the position of the probe is tracked in order to determine the position at which the measurement was taken. As outlined in step 403 the position of the probe tip or measurement component 26 may be known relative to the probe marker 24. An RGB camera 6 on the headset 3 may capture an image of the probe marker 24. Coordinates may be assigned to the probe tip 26 within a coordinate system (headset coordinate system). This may be achieved through detection algorithms, such as image pattern recognition. The coordinates are sent to the processor which creates a virtual map highlighting regions of cancerous tissue.

At step 409 one or more biological landmarks are tracked to determine their position at each point in time during the surgical procedure. The use of biological landmarks provides an approach of tracking the patient throughout the procedure without the need to use physical markers.

The tracking step 409 may involve a number of sub steps. As outlined above, the biological landmarks (such as blood vessels) may be tracked through the biological landmark emitting a fluorescent signal after being injected by a contrast agent during or before the surgical procedure. The NIR light source 8 on the headset 3 illuminates the region of patient tissue of interest at one or more wavelengths known to cause fluorescence. The emitted fluorescence light from the patient is captured by the NIR camera(s) 6. The signal may then be filtered to remove unwanted noise. The raw data images formed from the captured NIR light are corrected or rectified using the calibration parameters obtained during the calibration step 401.

Feature of the biological landmarks are then detected (i.e. identified) so that the features of the biological landmarks can provide easy identifiable reference points for tracking through image frames. These features may include edges or intersection points of the blood vessels, for instance. Robust algorithms are used to identify these features based on, for example, the size, shape and colour intensity of the biological landmarks. The features may be identified as 2D or 3D features. For instance, if multiple NIR cameras are used this may enable 3D model of each frame to be determined. However, if only a single camera is used (and no depth sensor) a 2D image may instead be used. The features from each frame are extracted. The position of the features is set within CS2.

The relative position between the features of the biological landmark and the probe measurement positions can be calculated to determine a relationship between their positions that can be used throughout the procedure to update the position of the virtual map on the display through tracking the positions of the features of the biological landmarks. This may be through transformations between the patient and headset coordinate systems (CS1) where the relative positions between them can readily determined.

The features are then tracked between consecutive frames to estimate the motion This is through finding correspondences between the 2D/3D features across consecutive frames taken at successive points in time.

In order to estimate the motion of the features of the biological landmarks across consecutive frames a transformation matrix is calculated. This may be a 2D or 3D transformation depending on whether the tracked features are 2D or 3D. A separate transformation may be calculated to estimate the motion between each consecutive frame, thus providing a frame to frame transformation. Owing to the fact that a patient's body is dynamic, with constant movement due to respiratory movements and deformation of soft tissues, the biological landmark features may be displaced across consecutive frames. In order to correct for this, non-rigid registration and elastic deformation correction may be used to estimate the position (in 2D or 3D) of the biological features in real-time. (These are preferred when compared to rigid body registration as for dynamic objects rigid body registration presents problems associated to sensitivity, repeatability, robustness or multiple views.) The elastic deformable models may be generated to help maximize the accuracy of the registration. Furthermore, the shape of the features can also change across frames due to this patient movement, which could result in non-uniformities when comparing the features. The displacement value of the features may be confirmed with image gradient operation on the 3D data. For this, a threshold level is assigned based on an acceptance criterion. Once that threshold is reached, the virtual map is registered as described below in section 411. For instance, this may be when there is around an 80 to 90% match of features. The transformation matrix may be continuously estimated throughout the surgical procedure to provide ongoing tracking of the biological markers.

Prior to calculating the transformation matrix, an optimisation procedure may be applied to remove noisy or incomplete data. This may involve using global optimization. Alternatively, or in addition, an outlier rejection algorithm or optimization filter may be used. For instance, optimized Random sample consensus (RANSAC) or Iterative closest point algorithms. Advantageously these techniques can help to remove noise and speckle thereby reducing false matches that may be caused by tracking drifts derived from noisy data.

Step 405 is done simultaneously to step 407, thus associating the measurements taken with the probe to the collected data coordinates. Steps 405 and 407 are also done simultaneously with step 409 such that the location of the probe measurements can be tracked relative to the features of the biological landmarks both when the measurements are taken and also throughout the rest of the surgical procedure.

At step 411 registration is performed such that the coordinates of the probe measurements can be mapped to the coordinates of the features of the biological landmarks. This ensures that the virtual map can be correctly aligned with the region of the patient's body when viewed on the display.

At step 412 the positions of the surgeon's eyes are tracked to determine their gaze relative to the display. The headset 3 may have one or more eye tracking sensor(s) located on it capable of tracking the position of the surgeon's eyes. This may be achieved through the eye tracking sensors tracking points on the surgeon's retina. The IPD (inter pupillary distance) of the user may also be used in conjunction with the position of the surgeon's eyes to ensure accurate placement of the measurements on the display.

One or more sensors may be used to track both of the surgeon's eyes. Alternatively, separate tracking sensor(s) may track the position of each eye. The eye tracking sensor may comprise an IR light source and detector that scans the position of each eye to determine its position. The light source may be in the form of an LED or laser. Alternatively, or in addition, the eye tracking sensor may be an electric potential eye tracking sensor. The electric potential eye tracking sensor uses electrodes placed around the eye to measure the movement of the eye.

At step 414 the probe measurements are displayed on the display overlaid with respect to the position of the patient where they were acquired. The processor displays the virtual map on the displays 4a 4b. Each display 4a 4b will display the virtual map positioned correctly relative to its associated eye of the surgeon and their field of view of the patient. The position, orientation and scale of the virtual map is dynamically adjusted in real-time based on the location of the AR headset 3 relative to the patient which is continually tracked. The surgeon wearing the AR headset perceives the virtual map as overlaid on the patient's body such that it can be used as visual guidance during surgery.

Step 409, 411, 412 and 414 are repeatedly carried out through the procedure to take into account the movement of the patient throughout the procedure ensuring that the measurements are overlaid on the display at the correct position.

Steps 405 and 407 may also optionally be repeatedly carried out to acquire additional measurements throughout the procedure.

In order to ensure that the virtual map is displayed at the correct position orientation and scale in real-time, the position of the headset relative to the target must be continually tracked during the procedure. As outlined above, this may be through tracking the position of the biological landmarks of the patient throughout the procedure. However, tracking the distance between the headset and the patient can also be carried out throughout the surgical procedure. This may be through one or more depth sensors located on the headset, such as time of flight sensors. For instance, the depth sensor may enable the position and/or orientation (pose) of the headset relative to the patient to be determined throughout the procedure. This may be through generating point clouds or depth images of the surgical site. This allows the scale of the image on the display to be adjusted accordingly to take into account distance from the patient. This may aid in providing a transformation between the patient coordinates and the headset coordinates. For instance, the depth sensor may enable the position and/or orientation (pose) of the headset relative to the patient to be determined throughout the procedure. This may add an additional parameter to the biological landmark tracking through knowing the distance between the biological landmark and the headset. As the probe data may be obtained as 3D data the use of point clouds or depth images may enable 3D data of the biological landmark features to be obtained, such that they can be compared to determine a relationship between the two positions.

The processor may be a processor for executing instructions in a data processing device. Instructions may be stored in a memory, for example. The processor may include one or more processing units (e.g., in a multi-core configuration) for executing instructions. The instructions may be executed within a variety of different operating systems on the data processing device, such as UNIX, LINUX, Microsoft Windows^{®}, etc. More specifically, the instructions may cause various data manipulations on data stored in memory (e.g., create, read, update, and delete procedures). It should also be appreciated that upon initiation of a computer-implemented method, various instructions may be executed during initialization. Some operations may be required in order to perform one or more methods described herein, while other operations may be more general and/or specific to a particular programming language (e.g., C, C#, C++, Java, Python or other suitable programming languages, etc.).

Having described aspects of the disclosure in detail, it will be apparent that modifications and variations are possible without departing from the scope of aspects of the disclosure as defined in the appended claims. As various changes could be made in the above constructions, products, and methods without departing from the scope of aspects of the disclosure, it is intended that all matter contained in the above description and shown in the accompanying drawings shall be interpreted as illustrative and not in a limiting sense.

Alternative methods can be used to track the position of the probe other than the procedure described above in step 407 of using an RGB camera 10 on the headset and a visible marker on the probe. For instance, the infrared camera (or NIR) may be used for tracking instead. This may be through the probe marker emitting an infrared signal which is detected by the probe. Alternatively, any type of electromagnetic emitter (transmitter) and sensor may be used to track the probe position. Alternatively, or in addition, machine learning based custom trained model may be used.

The probe tracking described above is described as being carried out by sensors on the headset. Alternatively, the sensors 6, 10 (e.g. cameras) may be located separate to the headset 3. In this arrangement, a marker on the headset 3 is also tracked to ensure the position of the headset 3 is also known with respect to the external tracking system. In other arrangements, the probe 22 may also, or instead, include one or more sensors that enable it to track its own position and communicate this to the processor. For instance, the probe 22 may comprise an inertial measurement unit (IMU) enabling details of its position and/or orientation to be tracked.

Alternatively, the probe may be tracked by using an electromagnetic field generated under, or in the close vicinity of the patient and operating scene. In this arrangement, the probe and headset comprise magnetic markers placed on them. In this way, the magnetic field generated by said mat detects the movement of these small magnetic units within the electromagnetic field thereby tracking their motion to obtain their spatial localisation. In this arrangement, a RGB camera used to track the probe may not be required.

The sensors that track the biological marker may also be located external to the headset 3 in the same way as described above. The light source 8 that illuminates the biological landmark to perform fluorescence may be located remote from the AR headset 3, rather than specifically being located on said headset 3.

In some arrangements, the processor and its associated memory may be located on the AR headset. In other arrangements, the processor and memory may be located remote to the AR headset. When located on the headset the processor may be located at the back of the head of the wearer. In other arrangements, the processor may be located in the housing of headset (i.e. the part in front of the wearer's head). However, any other location of the processor may be possible.

Although the above description focuses on Raman spectroscopy, the above system and methods might also be used for other purposes. For instance, the probe is not necessary limited to being a Raman probe. The probe could alternatively, be a temperature probe capable of acquiring temperature measurements from a patient. The temperature measurements may be displayed on the display as a virtual map at the correct position in the surgeon's view relative to where they were acquired. The probe 22 may be used to take measurements from any region of the patient's body. For instance, this may be the brain's cortex with the biological landmark a blood vessel pattern on the brain surface.

Alternatively, it may be looking at the patient's liver with the biological landmark being a blood vessel pattern near to the liver. The surgical procedure described above is for cancer surgery, i.e. for the removal of the cancerous tissue. The virtual map aids to guide the surgeon during said surgery. However, it would be understood that the above systems may be used for any type of surgery. In other arrangements, the probe may be an electromagnetic probe. For instance, the electromagnetic probe may be an electromagnetic pulsation probe. The electromagnetic pulsation probe may be used to take measurements from or evaluate a patient's brain functions in an invasive procedure. Alternatively, any other type of probe may be used, such as for instance a PH probe. In other arrangements, the probe may be used to place virtual marks on the face of a user to create a virtual map that can be later used during surgery.

The term surgeon is used herein to denote the person wearing the AR headset 3. However, as would be understood the person need not necessarily be a surgeon and could be any person using the device. The terms surgeon, wearer and user may be interchangeably used herein. The term coordinate system used herein may be understood to mean coordinate system.

The detection of the image and associated actions are described as being performed by a camera. However, any type of image sensor/image sensing device may be used. The camera may be configured to detected still images or videos.

The AR headset is shown in the Figures as having two displays attached to a housing with two arms (temples). However, it will be understood that the AR headset of the present invention is not limited to such an arrangement, and any conventional means of attaching a head mounted display (HMD) to a wearer could be envisaged. This may include using straps that pass around and/or over the head to hold the headset in place. Alternatively, an attachment means that attaches the device over the whole of the top of the head, such as a hat may be used. In addition, the particular arrangement of the housing shown is just an example and any type of housing may be used. In other arrangements, rather than an AR headset the AR system may comprise one or more glass displays that can be placed over the patient. The above methods thereby used to display the measurements on said display as described in the embodiments above.

The step of using eye tracking as described herein provides increased accuracy of aligning the virtual map on the display. However, this is an optional step and suitable alignment may be achieved without using eye tracking. In addition, the eye tracking sensors need not necessarily be located on the AR headset 3 and may be located within an external sensing system that is separate from the headset 3.

Although NIR cameras and NIR sources for carrying out the fluorescent based procedures have been described herein it is not necessarily limited to such wavelength bands. Light of other wavelengths may be used. For instance, any range of infrared wavelengths may be used. In other arrangements, visible light may be used. For instance, IR light may be used to see blood vessels without the need to inject fluorescent dye.

It would be understood that the type and number of cameras and sensors as described above are not limiting. Single or multiple cameras and/or distance sensors may be used. A motion tracker, with a time of flight, and a stereoscopic camera system may be one such example to provide an accurate positioning.

In the above described methods the features of the biological landmark 30 may be used as the primary reference when determining the position of the probe 22 relative to the biological landmark 30. However, it would be understood, alternatively, the probe position may be used as the primary reference point rather than the features of the biological landmark 30.

In other arrangements, rather than using a biological landmark an artificial marker on the surgical table may be used. This may also be used in addition to the biological landmark to improve accuracy of tracking.

Biological landmarks are described as being tracked in the methods herein. It may be understood that this tracking may also be tracking of the target tissue (i.e. rather than blood vessels and the other described examples).

The sensors described in relation to Figures 2 and 3 are only an example of the type and number of sensors that may be utilised. In many arrangements, multiple sensors may be used to maximize the accuracy of the probe and patient tracking.

As shown in the above examples there may be two displays 4a 4b. However, in other arrangements, a single display for both eyes may be used. Alternatively, the AR headset may comprise a single near eye display over a single eye, i.e. only having 4a and/or 4b, rather than both.

Although it is shown above that data may be communicated between different components through one or more cables it would be understood that the connections need not necessarily be wired. For instance, any type of wireless communication means may be used, for instance, to transmit signals from the probe, headset and processor. This may include USB, Wi-Fi, or Bluetooth for instance.

In some arrangements, medical images that have been previously acquired may also be included on the virtual map. For instance, this may be data from scans of the patient, such as CT or MRI scans. In addition, additional information in the field of view may be displayed in addition to the virtual map. For example, the patient specific information. This may be information from the patient's file. The AR headset may also be configured to implement other features such as having a communication means capable of providing audio or video signals to the surgeon such that they can perform a telementoring process.

In other alternative arrangements, additional applications may be foreseen and would allow to register the virtual map with patient-specific digital data (i.e., patient-specific 3D models of high-risk anatomical structures surrounding areas of cancerous tissue). This may be achieved by:
**▪** Using sensors integrated in the AR headset that detect a biological marker. The data collected by the sensors is used to create a patient-specific 3D model of this structure based on the biological marker (e.g. the biological marker may indicate high risk anatomical structures that are to be displayed in addition to the virtual map). The system matches the 3D model coordinate system to patient coordinate system for use in the procedures as described above i.e. displaying both the measurements that form the 3D model and the probe measurements.
▪ Attaching a physical marker to the patient body and scanning the patient. Then, patient-specific 3D models are obtained from the medical scans and their position relative to the marker is imported into the system. The 3D models coordinate system is matched to patient coordinate system. In this way, the patient scans (such as MRI CT , ultrasound), and/or camera measurements can be displayed in addition to the probe measurements.

## Claims

1. An augmented reality (AR) system (100) for obtaining and displaying measurements from a patient during a medical procedure, the system comprising:
an AR headset (2) comprising a near eye display (4a, 4b) for displaying the measurements overlaid in a surgeon's view of the patient's tissue (28) at a position with respect to the patient to which they were collected;
at least one sensor (18) for tracking the location of the patient;
a memory for storing the measurements and their associated positions; and
a processor;
wherein the system is configured to perform the following steps during the medical procedure:
(a) receiving measurements collected from the patient's tissue (28), wherein the measurements are collected by a probe (22) being positioned adjacent to various regions of the patient's tissue (28);
(b) tracking, the probe's (22) position whilst the probe (22) is collecting measurements to determine a position at which the measurements are collected;
(c) simultaneously to performing steps (a) and (b): tracking, using the at least one sensor, a position of a biological landmark (30) on the patient such that a relationship can be determined between the position of the biological landmark (30) and the position at which the measurements are collected;
(d) displaying on the near eye display (4a, 4b) the measurements at a position, such that the measurements are aligned in the surgeon's view of the patient's tissue (28) at the position with respect to the patient to which they were collected, throughout the medical procedure, by:
continuously tracking the position of the biological landmark (30) throughout the medical procedure to obtain its current position;
updating the position of the measurements on the display based on the biological landmark's (30) current position.

2. The AR system (100) of claim 1, further comprising creating a virtual map of the measurements, wherein displaying on the near eye display (4a, 4b) the measurements comprises displaying the virtual map.

3. The AR system (100) of claim 1 or 2, wherein tracking the position of the biological landmark (30) on the patient further comprises: illuminating a region of the patient using light of a specific wavelength and detecting by the sensor a fluorescent signal emitted by the biological landmark (30) in response to the illuminating.

4. The AR system (100) of any preceding claim, further comprising a probe (22).

5. The AR system (100) of claim 4, wherein the probe (22) is a Raman probe.

6. The AR system (100) of any preceding claim, wherein one or more features of the biological landmark (30) is tracked.

7. The AR system (100) of claim 6, wherein continuously tracking the position of the features throughout the medical procedure to obtain its current position further comprises:
capturing, by the sensor, images of the patient;
extracting the one or more features of the biological landmark (30) from the images;
determining a correspondence between the one or more features across consecutive frames of the images;
estimating motion of the one or more features across consecutive frames of the images by using a transformation matrix.

8. The AR system (100) of claim 7, wherein estimating motion of the one or more features across consecutive frames of images by using a transformation matrix further comprises: applying a rigid or non-rigid registration.

9. The AR system (100) of claim 6, 7, or 8 wherein one or more features of the biological landmark (30) comprise edges or points of intersection of the biological landmark (30).

10. The AR system (100) of any preceding claim, wherein the biological landmark (30) comprises at least one of blood vessels of the patient, Lymph nodes of the patient, nervous systems of the patient, organ surfaces of the patient.

11. The AR system (100) of any preceding claim, wherein the step of tracking the probe's (22) position comprises:
detecting, via the sensor, a marker on the probe (22) and calculating a position of a tip of the probe (22) representing the position at which the measurement is collected; or
detecting a change in magnetic field caused by the movement of a magnetic marker on the probe (22), wherein the change in magnetic field identifies the position of the probe (22).

12. The AR system (100) of any preceding claim, wherein the relationship between the position of the biological landmark (30) and the position at which the measurements are collected is determined by transforming the position of the biological landmark (30) and the position at which the measurements are collected into a common coordinate system.

13. The AR system (100) of any preceding claim, wherein displaying on the near eye display (4a, 4b) the measurements at a position, such that the measurements are aligned in the surgeon's view of the patient's tissue (28) at the position with respect to the patient to which they were collected, throughout the medical procedure, further comprises:
tracking a position of the surgeon's eyes and adjusting the position of the measurements on the near eye display (4a, 4b) based on the current position of the surgeon's eyes.

14. A computer program product comprising instructions which, when the program is executed by a computer, cause the computer to carry out the steps of:
(a) receiving measurements collected from a patient's tissue (28), wherein the measurements are collected by a probe (22) being positioned adjacent to various regions of the patient's tissue (28);
(b) tracking, the probe (22)'s position whilst the probe (22) is collecting measurements to determine a position at which the measurements are collected;
(c) simultaneously to performing steps (a) and (b): tracking, using the at least one sensor, a position of a biological landmark (30) on the patient such that a relationship can be determined between the position of the biological landmark (30) and the position at which the measurements are collected;
(d) displaying on a near eye display (4a, 4b) of an AR headset (2) worn by the surgeon the measurements at a position, such that the measurements are aligned in the surgeon's view of the patient's tissue (28) at the position with respect to the patient to which they were collected, throughout the medical procedure, by:
continuously tracking the position of the biological landmark (30) throughout the medical procedure to obtain its current position;
updating the position of the measurements on the near eye display (4a, 4b) based on the position of the biological landmark's current position.

## Patentansprüche

1. Erweitertes Realitäts- (AR-) System (100) zum Erhalten und Anzeigen von Messungen von einem Patienten während eines medizinischen Eingriffs, wobei das System Folgendes umfasst:
ein AR-Headset (2) umfassend eine augennahe Anzeige (4a, 4b) zum Anzeigen der Messungen, die in das Sichtfeld eines Chirurgen auf das Gewebe (28) des Patienten an einer Position in Bezug auf den Patienten, an der sie erfasst wurden, eingeblendet werden;
mindestens einen Sensor (18) zum Verfolgen des Standorts des Patienten;
einen Speicher zum Speichern der Messungen und ihrer zugehörigen Positionen; und
einen Prozessor;
wobei das System so konfiguriert ist, dass es während des medizinischen Eingriffs die folgenden Schritte ausführt:
(a) Empfangen von Messungen, die aus dem Gewebe (28) des Patienten erfasst wurden, wobei die Messungen von einer Sonde (22) erfasst werden, die neben verschiedenen Bereichen des Gewebes (28) des Patienten positioniert ist;
(b) Verfolgen der Position der Sonde (22), während die Sonde (22) Messungen erfasst, um eine Position zu bestimmen, an der die Messungen erfasst werden;
(c) gleichzeitig mit der Durchführung der Schritte (a) und (b): Verfolgen einer Position eines biologischen Orientierungspunkts (30) an dem Patienten unter Verwendung des mindestens einen Sensors, so dass eine Beziehung zwischen der Position des biologischen Orientierungspunkts (30) und der Position, an der die Messungen erfasst werden, bestimmt werden kann;
(d) Anzeigen der Messungen auf der augennahen Anzeige (4a, 4b) an einer Position, so dass die Messungen in dem Sichtfeld des Chirurgen während des medizinischen Eingriffs auf das Gewebe (28) des Patienten an der Position in Bezug auf den Patienten, an der sie erfasst wurden, ausgerichtet sind, durch:
kontinuierliches Verfolgen der Position des biologischen Orientierungspunkts (30) während des gesamten medizinischen Eingriffs, um dessen aktuelle Position zu erhalten;
Aktualisieren der Position der Messungen auf dem Display basierend auf der aktuellen Position des biologischen Orientierungspunkts (30).

2. AR-System (100) nach Anspruch 1, weiter umfassend das Erstellen einer virtuellen Karte der Messungen, wobei das Anzeigen der Messungen auf der augennahen Anzeige (4a, 4b) das Anzeigen der virtuellen Karte umfasst.

3. AR-System (100) nach Anspruch 1 oder 2, wobei das Verfolgen der Position des biologischen Orientierungspunkts (30) auf dem Patienten weiter Folgendes umfasst: Beleuchten eines Bereichs des Patienten mit Licht einer bestimmten Wellenlänge und Detektieren eines Fluoreszenzsignals, das von dem biologischen Orientierungspunkt (30) als Reaktion auf das Beleuchten emittiert wird, durch den Sensor.

4. AR-System (100) nach einem vorstehenden Anspruch, weiter umfassend eine Sonde (22).

5. AR-System (100) nach Anspruch 4, wobei die Sonde (22) eine Raman-Sonde ist.

6. AR-System (100) nach einem vorstehenden Anspruch, wobei ein oder mehrere Merkmale des biologischen Orientierungspunkts (30) verfolgt werden.

7. AR-System (100) nach Anspruch 6, wobei das kontinuierliche Verfolgen der Position der Merkmale während des gesamten medizinischen Eingriffs, um deren aktuelle Position zu erhalten, weiter Folgendes umfasst:
Aufnehmen von Bildern des Patienten durch den Sensor;
Extrahieren des einen Merkmals oder der mehreren Merkmale des biologischen Orientierungspunkts (30) aus den Bildern;
Bestimmen einer Übereinstimmung zwischen dem einen oder den mehreren Merkmalen über aufeinanderfolgende Bildrahmen hinweg;
Schätzen der Bewegung des einen oder der mehreren Merkmale über aufeinanderfolgende Bildrahmen hinweg unter Verwendung einer Transformationsmatrix.

8. AR-System (100) nach Anspruch 7, wobei das Schätzen der Bewegung des einen oder der mehreren Merkmale über aufeinanderfolgende Bildrahmen hinweg unter Verwendung einer Transformationsmatrix weiter Folgendes umfasst: Anwenden einer starren oder nicht starren Registrierung.

9. AR-System (100) nach Anspruch 6, 7, oder 8, wobei ein oder mehrere Merkmale des biologischen Orientierungspunkts (30) Kanten oder Schnittpunkte des biologischen Orientierungspunkts (30) umfassen.

10. AR-System (100) nach einem vorstehenden Anspruch, wobei der biologische Orientierungspunkt (30) mindestens eines von Blutgefäßen des Patienten, Lymphknoten des Patienten, Nervensystemen des Patienten, Organoberflächen des Patienten umfasst.

11. AR-System (100) nach einem vorstehenden Anspruch, wobei der Schritt des Verfolgens der Position der Sonde (22) Folgendes umfasst:
Detektieren eines Markers auf der Sonde (22) über den Sensor und Berechnen einer Position einer Spitze der Sonde (22), die die Position darstellt, an der die Messung erfasst wird; oder
Detektieren einer Änderung des Magnetfelds, die durch die Bewegung eines magnetischen Markers auf der Sonde (22) verursacht wird, wobei die Änderung des Magnetfelds die Position der Sonde (22) identifiziert.

12. AR-System (100) nach einem vorstehenden Anspruch, wobei die Beziehung zwischen der Position des biologischen Orientierungspunkts (30) und der Position, an der die Messungen erfasst werden, durch Transformieren der Position des biologischen Orientierungspunkts (30) und der Position, an der die Messungen erfasst werden, in ein gemeinsames Koordinatensystem bestimmt wird.

13. AR-System (100) nach einem vorstehenden Anspruch, wobei das Anzeigen der Messungen auf der augennahen Anzeige (4a, 4b) an einer Position, so dass die Messungen in dem Sichtfeld des Chirurgen auf das Gewebe (28) des Patienten an der Position in Bezug auf den Patienten, an der sie erfasst wurden, während des gesamten medizinischen Eingriffs ausgerichtet sind, weiter Folgendes umfasst:
Verfolgen einer Position der Augen des Chirurgen und Anpassen der Position der Messungen auf der augennahen Anzeige (4a, 4b) basierend auf der aktuellen Position der Augen des Chirurgen.

14. Computerprogrammprodukt, das Anweisungen umfasst, die, wenn das Programm von einem Computer ausgeführt wird, den Computer veranlassen, die folgenden Schritte auszuführen:
(a) Empfangen von Messungen, die aus dem Gewebe (28) eines Patienten erfasst wurden, wobei die Messungen durch eine Sonde (22) erfasst werden, die benachbart zu verschiedenen Bereichen des Gewebes (28) des Patienten positioniert ist;
(b) Verfolgen der Position der Sonde (22), während die Sonde (22) Messungen erfasst, um eine Position zu bestimmen, an der die Messungen erfasst werden;
(c) gleichzeitig mit der Durchführung der Schritte (a) und (b): Verfolgen einer Position eines biologischen Orientierungspunkts (30) an dem Patienten unter Verwendung des mindestens einen Sensors, so dass eine Beziehung zwischen der Position des biologischen Orientierungspunkts (30) und der Position, an der die Messungen erfasst werden, bestimmt werden kann;
(d) Anzeigen der Messungen an einer Position auf einer augennahen Anzeige (4a, 4b) eines von dem Chirurgen getragenen AR-Headsets (2), so dass die Messungen während des gesamten medizinischen Eingriffs in dem Sichtfeld des Chirurgen auf das Gewebe (28) des Patienten an der Position ausgerichtet sind, an der sie in Bezug auf den Patienten erfasst wurden, durch:
kontinuierliches Verfolgen der Position des biologischen Orientierungspunkts (30) während des gesamten medizinischen Eingriffs, um dessen aktuelle Position zu erhalten;
Aktualisieren der Position der Messungen auf der augennahen Anzeige (4a, 4b) basierend auf der Position der aktuellen Position des biologischen Orientierungspunkts.

## Revendications

1. Système de réalité augmentée, RA, (100) permettant d'obtenir et d'afficher des mesures d'un patient au cours d'un acte médical, le système comprenant :
un casque RA (2) comprenant un dispositif d'affichage proche des yeux (4a, 4b) pour afficher les mesures superposées dans une vue du chirurgien des tissus du patient (28) à une position par rapport au patient à laquelle elles ont été recueillies ;
au moins un capteur (18) permettant de suivre l'emplacement du patient ;
une mémoire pour stocker les mesures et leurs positions associées ; et
un processeur ;
dans lequel le système est configuré pour effectuer les étapes suivantes au cours de l'acte médical :
(a) la réception de mesures recueillies à partir des tissus du patient (28), dans lequel les mesures sont recueillies par une sonde (22) qui est positionnée à proximité de diverses régions des tissus du patient (28) ;
(b) le suivi de la position de la sonde (22) pendant que la sonde (22) recueille des mesures afin de déterminer une position à laquelle les mesures sont recueillies ;
(c) simultanément à l'exécution des étapes (a) et (b) : le suivi, à l'aide de l'au moins un capteur, d'une position d'un repère biologique (30) sur le patient de manière à pouvoir déterminer une relation entre la position du repère biologique (30) et la position à laquelle les mesures sont recueillies ;
(d) l'affichage sur le dispositif d'affichage proche des yeux (4a, 4b) des mesures à une position, de telle sorte que les mesures soient alignées dans la vue du chirurgien des tissus du patient (28) à la position par rapport au patient à laquelle elles ont été recueillies, tout au long de l'acte médical, par :
le suivi continu de la position du repère biologique (30) tout au long de l'acte médical afin d'obtenir sa position actuelle ;
la mise à jour de la position des mesures sur le dispositif d'affichage sur la base de la position actuelle du repère biologique (30).

2. Système RA (100) selon la revendication 1, comprenant en outre la création d'une carte virtuelle des mesures, dans lequel l'affichage des mesures sur le dispositif d'affichage proche des yeux (4a, 4b) comprend l'affichage de la carte virtuelle.

3. Système RA (100) selon la revendication 1 ou la revendication 2, dans lequel le suivi de la position du repère biologique (30) sur le patient comprend en outre : l'éclairement d'une région du patient à l'aide d'une lumière d'une longueur d'onde spécifique et la détection par le capteur d'un signal fluorescent émis par le repère biologique (30) en réponse à l'éclairement.

4. Système RA (100) selon une quelconque revendication précédente, comprenant en outre une sonde (22).

5. Système RA (100) selon la revendication 4, dans lequel la sonde (22) est une sonde Raman.

6. Système RA (100) selon une quelconque revendication précédente, dans lequel une ou plusieurs caractéristiques du repère biologique (30) sont suivies.

7. Système RA (100) selon la revendication 6, dans lequel le suivi continu de la position des caractéristiques tout au long de l'acte médical pour obtenir leur position actuelle comprend en outre :
la capture, par le capteur, d'images du patient ;
l'extraction de la ou des caractéristiques du repère biologique (30) à partir des images ;
la détermination d'une correspondance entre la ou les caractéristiques sur des trames consécutives des images ;
l'estimation du mouvement de la ou des caractéristiques sur des trames consécutives des images à l'aide d'une matrice de transformation.

8. Système RA (100) selon la revendication 7, dans lequel l'estimation du mouvement de la ou des caractéristiques sur des trames consécutives d'images à l'aide d'une matrice de transformation comprend en outre : l'application d'un enregistrement rigide ou non rigide.

9. Système RA (100) selon la revendication 6, la revendication 7, ou la revendication 8 dans lequel une ou plusieurs caractéristiques du repère biologique (30) comprennent des arêtes ou des points d'intersection du repère biologique (30).

10. Système RA (100) selon une quelconque revendication précédente, dans lequel le repère biologique (30) comprend au moins l'un parmi des vaisseaux sanguins du patient, des ganglions lymphatiques du patient, des systèmes nerveux du patient, des surfaces d'organes du patient.

11. Système RA (100) selon une quelconque revendication précédente, dans lequel l'étape de suivi de la position de la sonde (22) comprend :
la détection, au moyen du capteur, d'un marqueur sur la sonde (22) et le calcul d'une position d'une pointe de la sonde (22) représentant la position à laquelle la mesure est recueillie ; ou
la détection d'un changement de champ magnétique causé par le mouvement d'un marqueur magnétique sur la sonde (22), dans lequel le changement de champ magnétique identifie la position de la sonde (22).

12. Système RA (100) selon une quelconque revendication précédente, dans lequel la relation entre la position du repère biologique (30) et la position à laquelle les mesures sont recueillies est déterminée en transformant la position du repère biologique (30) et la position à laquelle les mesures sont recueillies en un système de coordonnées commun.

13. Système RA (100) selon une quelconque revendication précédente, dans lequel l'affichage sur le dispositif d'affichage proche des yeux (4a, 4b) des mesures à une position, de telle sorte que les mesures soient alignées dans la vue du chirurgien des tissus du patient (28) à la position par rapport au patient à laquelle elles ont été recueillies, tout au long de l'acte médical, comprend en outre :
le suivi d'une position des yeux du chirurgien et l'ajustement de la position des mesures sur le dispositif d'affichage proche des yeux (4a, 4b) sur la base de la position actuelle des yeux du chirurgien.

14. Produit de programme informatique comprenant des instructions qui, lorsque le programme est exécuté par un ordinateur, amènent l'ordinateur à effectuer les étapes consistant à :
(a) recevoir des mesures recueillies à partir des tissus d'un patient (28), dans lequel les mesures sont recueillies par une sonde (22) qui est positionnée à proximité de diverses régions des tissus du patient (28) ;
(b) le suivi de la position de la sonde (22) pendant que la sonde (22) recueille des mesures afin de déterminer une position à laquelle les mesures sont recueillies ;
(c) simultanément à l'exécution des étapes (a) et (b) : le suivi, à l'aide de l'au moins un capteur, d'une position d'un repère biologique (30) sur le patient de manière à pouvoir déterminer une relation entre la position du repère biologique (30) et la position à laquelle les mesures sont recueillies ;
(d) l'affichage sur un dispositif d'affichage proche des yeux (4a, 4b) d'un casque RA (2) porté par le chirurgien des mesures à une position, de telle sorte que les mesures soient alignées dans la vue du chirurgien des tissus du patient (28) à la position par rapport au patient à laquelle elles ont été recueillies, tout au long de l'acte médical, par :
le suivi en continu de la position du repère biologique (30) tout au long de l'acte médical afin d'obtenir sa position actuelle ;
la mise à jour de la position des mesures sur le dispositif d'affichage proche des yeux (4a, 4b) sur la base de la position actuelle du repère biologique.
